# EUROPEAN PATENT SPECIFICATION

(11) **EP 2 379 169 B1**
(45) Date of publication and mention of the grant of the patent: **15.07.2015**
(21) Application number: 08876273.7
(22) Date of filing: 17.12.2008
(51) Int. Cl.: A61N 1/362, A61N 1/365, A61B 5/053, A61B 5/08, A61B 5/11, A61N 1/368, A61N 1/372

(54) **IMPLANTABLE MEDICAL DEVICE FOR MONITORING SYNCHRONICITY OF THE VENTRICLES OF A HEART**
IMPLANTIERBARE MEDIZINISCHE VORRICHTUNG ZUR ÜBERWACHUNG DER SYNCHRONIZITÄT DER HERZKAMMERN
DISPOSITIF MÉDICAL IMPLANTABLE DE SURVEILLANCE DE SYNCHRONICITÉ DES VENTRICULES D'UN COEUR

(43) Date of publication of application: 26.10.2011
(73) Proprietor: St. Jude Medical AB, 175 84 Järfälla (SE)
(72) Inventor: HOLMSTRÖM, Nils, 175 57 Järfälla (SE); LJUNGSTRÖM, Karin, 165 70 Hässelby (SE); BROOME, Michael, 178 38 Ekerö (SE); EMANUELSSON, Cecilia, 195 92 Märsta (SE)
(74) Representative: Sjölander, Henrik
(86) International application number: PCT/SE2008/000720
(87) International publication number: WO 2010/071488

(56) References cited:
- WO-A-2007/149018
- WO-A-2008/133552
- US-A1- 2001 012 953
- US-A1- 2004 078 058
- US-A1- 2008 004 667
- US-A1- 2008 234 773
- US-A1- 2008 249 583
- US-A1- 2008 294 216
- US-B1- 6 751 503

## Description

### Technical field

The present invention generally relates to the field of implantable heart stimulation devices, such as pacemakers, and similar cardiac stimulation devices that also are capable of monitoring and detecting electrical activities and events within the heart. More specifically, the present invention relates to an implantable medical device for monitoring ventricular synchronicity of a heart.

### Background art

Implantable heart stimulators that provide stimulation pulses to selected locations in the heart, e.g. selected chambers have been developed for the treatment of cardiac diseases and dysfunctions. Heart stimulators have also been developed that affect the manner and degree to which the heart chambers contract during a cardiac cycle in order to promote the efficient pumping of blood.

Furthermore, the heart will pump more effectively when a coordinated contraction of both atria and ventricles can be provided. In a healthy heart, the coordinated contraction is provided through conduction pathways in both the atria and the ventricles that enable a very rapid conduction of electrical signals to contractile tissue throughout the myocardium to effectuate the atrial and ventricular contractions. If these conduction pathways do not function properly, a slight or severe delay in the propagation of electrical pulses may arise, causing asynchronous contraction of the ventricles which would greatly diminish the pumping efficiency of the heart. Patients, who exhibit pathology of these conduction pathways, such as patients with bundle branch blocks, etc., can thus suffer from compromised pumping performance. For example, asynchronous movements of the valve planes of the right and left side of the heart, e.g. an asynchronous opening and/or closure of the aortic and pulmonary valves, is such an asynchrony that affects the pumping performance in a negative way. This may be caused by right bundle branch block (RBBB), left bundle branch block (LBBB), or A-V block. In a well functioning heart, the left and right side of the heart contract more or less simultaneously starting with the contraction of the atria flushing down the blood through the valves separating the atria from the ventricles, in the right side of the heart through the tricuspid valve and in the left side of the heart through the mitral valve. Shortly after the atrial contraction the ventricles contract, which results in an increasing blood pressure inside the ventricles that first closes the one way valves to the atria and after that forces the outflow valves to open. In the right side of the heart it is the pulmonary valves that separate the right ventricle from the pulmonary artery that leads the blood to the lung, which is opened. In the left side of the heart the aortic valve separates the left ventricle from the aorta that transports blood to the whole body. The outflow valves, the pulmonary valve and aortic valve, open when the pressure inside the ventricle exceeds the pressure in the pulmonary artery and aorta, respectively. The ventricles are separated by the intra-ventricular elastic septum. Hence, for a well functioning heart a substantially synchronous operation of the left and right hand side of the heart, e.g. a synchronous opening and/or closure of the aortic and pulmonary, is of a high importance.

When functioning properly, the heart maintains its own intrinsic rhythm. However, patients suffering from cardiac arrhythmias, i.e. irregular cardiac rhythms, and/or from poor spatial coordination of heart contractions often need assistance in form of a cardiac function management system to improve the rhythm and/or spatial coordination of the heart contractions. Such systems are often implanted in the patient and deliver therapy to the heart, such as electrical stimulation pulses that evoke or coordinate heart chamber contractions. Thus, implantable heart stimulators that provide stimulation pulses to selected locations in the heart, e.g. selected chambers, have been developed for the treatment of cardiac diseases and dysfunctions. Heart stimulators have also been developed that affect the manner and degree to which the heart chambers contract during a cardiac cycle in order to promote the efficient pumping of blood.

In particular, various prior art procedures have been developed for addressing disorders related to asynchronous function of the heart. For instance, cardiac resynchronization therapy (CRT) can be used for effectuating synchronous atrial and/or ventricular contractions. Furthermore, cardiac stimulators may be provided that deliver stimulation pulses at several locations in the heart simultaneously, such as biventricular stimulators. For example, patients with heart failure symptoms and dyssynchronized cardiac chambers are often offered such a CRT device that synchronizes the right and left ventricle to obtain an improved cardiac functional performance and quality of life. The CRT settings should be optimized in terms of W interval and AV interval for optimized pumping performance. In the majority of CRT patients this optimizing of CRT parameters is normally performed at implant and perhaps at one regular follow-up. Ideally, this optimization should be performed more frequently to match the actual need of the patient.

Information about the mechanical functioning of a heart can be obtained by means electrical signals produced by the heart. In a healthy heart the sinus node, situated in the right atrium, generates electrical signals which propagates throughout the heart and control its mechanical movement. Some medical conditions, however, affect the relationship between the electrical and mechanical activity of the heart and, therefore, measurements of the electrical activity only cannot be relied upon as indicative of the true status of the heart or as suitable for triggering stimulation of the heart.

Consequently, there is a need within the art of methods and devices for obtaining accurate and reliable signals reflecting different aspects of mechanical functioning of the heart.

Impedance measurements, e.g. of the intra-cardiac impedance, has been shown to provide reliable information regarding the mechanical functioning of the heart. For example, through the impedance measurements, blood volume changes are detectable. Blood has a higher conductivity (lower impedance) than myocardial tissue and lungs. The relationship between impedance-volume is inverse, i.e. the more blood - the smaller impedance.

In US 2007/0066905, a system for optimizing a cardiac synchronization based on measure impedance signals is shown. In one embodiment of the system, the left ventricular impedance is measured, which reflects the contraction and expansion of the left ventricle. The obtained impedance signals are used to compute the impedance-indicated peak-to-peak volume indication of the left ventricle and/or an impedance-indicated maximum rate of change in the left ventricular volume. These parameters are then used to control a cardiac resynchronization. In US 2007/0271119 a similar optimization system is described. US2008/0234773 shows an implantable medical device, a system and a pacing analyzer according to the preamble of claims 1, 9 and 11, respectively.

Furthermore, in US 7,330,759 a cardiac pacemaker for bi-ventricular stimulation where impedance signals is used to obtain a synchronization of the left and right ventricles is shown. In particular, the second derivative of the intra-cardiac impedance pattern of a cardiac cycle is determined and maximized. This is based on the assumption that the intra-cardiac impedance pattern respectively reflects the volume of blood in a heart, the maximum acceleration to which the blood is subjected to in the heart is to be gauged from the maximum of the second derivative of that intra-cardiac impedance pattern, which value is correlated to contractility of the left ventricle.

However, the parameters used for the optimization in the prior art is often dependent on the physiological system including, inter alia, the heart and the vascular system which, for example, may entail that a response to a change of the stimulation parameters in terms of a change of a monitored parameter will be able to detect with a delay. This may, for example, lead to an overcompensation of the stimulation parameters. Furthermore, it cannot be ascertained that the monitored parameters reflect only the hemodynamical performance of the heart, which, in turn, may lead to a stimulation parameter setting that in the long-term is not optimal with respect to the hemodynamic performance of the heart.

In order to be able to optimize the functioning of the heart it is of paramount interest to obtain information that may provide a complete picture of the mechanical functioning and the pumping action of the heart and that provides accurate and reliable information of the mechanical functioning and the pumping action of the heart.

Moreover, in order to be able to optimize the functioning of the heart it is also of paramount interest to obtain information that may enable a fast and reliable optimization of the hemodynamic performance of the heart and, in particular, a fast and reliable synchronization of the ventricles, i.e. a coordinated contraction of the ventricles.

In addition, in order to be able to optimize the functioning of the heart it is further of high interest to obtain information that enables, in the long-term, an optimal synchronization of the ventricles, i.e. a coordinated contraction of the ventricles, with respect to the hemodynamic performance of the heart.

### Summary of the invention

An object of the present invention is to present an improved device for obtaining information that reflects the mechanical functioning and the pumping action of the heart, and, in particular, that reflects the synchronicity of the ventricle contractions in an accurate and reliable manner.

Another object of the present invention is to provide a device capable of monitoring a ventricular synchronicity of the heart in an accurate and reliable way.

These and other objects of the present invention are achieved by the devices as claimed in the independent claims. Further embodiments are defined in the dependent claims.

The present invention is based on the insight that certain characteristics of the morphology of the measured cardiogenic impedance reflect the septal movements or the mechanical movements of the septal wall. These characteristics may, in turn, be used to monitor ventricular dyssynchronicity. That is, certain features of the waveform morphology of the cardiogenic impedance reflect the mechanical movements of the septal wall, which movements are indicative of the synchronicity or dyssynchronicity of the ventricles of the heart. Asynchronous or dyssynchronous depolarization of the ventricles results in asynchronous myocardial contractions with regional dyskinetic cardiac tissue. The cardiac performance is very sensitive to small asynchronous cardiac movements, as the overall heart cycle is disturbed. One consequence is that not only the systolic part of the heart cycle will be less effective; the diastolic part (the filling part) will also be greatly tampered. In particular, the present invention is based on the insight that the cardiogenic impedance reaches a maximum value in connection with the end of the systolic phase of the ventricles and that a synchronized contraction of the ventricles is manifested in the impedance morphology by one pronounced impedance peak. An asynchrony in the functioning of the ventricles will, on the other hand, be reflected as a division of the impedance peak into (at least) two impedance peaks, which can be detected so as to monitor or identify the asynchrony of the contractions of the ventricles. These findings form the basis of the present invention and are utilized to construe a synchronicity measure which indicates a degree of synchronicity (or dyssynchronicity) between the right and left ventricle.

This approach for monitoring a synchronicity of the ventricles has a number of advantages. For example, since the information, i.e. the cardiogenic impedance morphology, used in the monitoring reflects the mechanical functioning and the pumping action of the heart, and, in particular, reflects the synchronicity of the ventricle contractions in an accurate and reliable manner, a device and method that are capable of monitoring a ventricular synchronicity of the heart in an accurate and reliable way can be achieved.

According to an embodiment of the present invention, points of maximum and/or minimum of the impedance signal morphology are detected in the predetermined time window, which time window corresponding to a systolic and diastolic phase of a cardiac cycle.

In an embodiment of the present invention, a first derivative of the impedance signal is calculated and points of local minima and/or local maxima of the first derivative are determined to be impedance peaks.

The implantable medical device according to preceding claims, further comprising a W delay determining unit adapted to initiate an optimization procedure, wherein the pace pulse generator is controlled to, based on the synchronicity index, iteratively adjust a W-interval so as to minimize the synchronicity index in the predetermined time window to obtain substantially synchronized ventricle contractions. Using information of the impedance peaks as basis for the determination of the synchronicity index also enables, in the long-term, an optimal synchronization of the ventricles, i.e. a coordinated contraction of the ventricles, with respect to the hemodynamic performance of the heart. Thus, the advantage of a fast and reliable optimization of the hemodynamic performance of the heart and, in particular, a fast and reliable synchronization of the ventricles, i.e. a coordinated contraction of the ventricles, can be achieved.

According to an embodiment of the present invention, the synchronicity is index based on a peak distance between two detected peaks in the differentiated impedance signal within the systole time window and the diastole time window. An increased peak distance corresponds to an increased value of the synchronicity index. For example, a synchronicity index for systole or diastole may be based on the difference between a local maximum point and a local minimum point. The higher difference between the respective peaks the higher synchronicity index. A higher synchronicity index indicates a higher degree of dyssynchronicity. During an optimization procedure, the W-interval that minimizes the synchronicity index is identified in an iterative procedure. Dependent on patient condition, the synchronization may be optimized during both systole and diastole. For example, one synchronicity index may be determined for systole and one index for diastole and the optimization may be performed on basis of a weighted total index based on the two indices associated with different weights, i.e. the weighted total index is minimized. Alternatively, the indices may be optimized separately to obtain a minimized index in systole and in diastole, respectively.

According to an embodiment of the present invention, the synchronicity index may be based on the number of detected peaks in differentiated impedance signal within the predetermined time window of systole and diastole, wherein an increased number of peaks corresponds to an increased value of the synchronicity index.

In an embodiment of the present invention, the synchronicity index may be based on a total peak area of one or several detected peaks of the differentiated impedance signal within the predetermined time window of systole and diastole measured above a predetermined threshold, wherein an increased peak area corresponds to an increased value of the synchronicity index. In a further alternative, the synchronicity index may be the total area of the peaks in both systole and diastole, or a weighted value of all peaks where different peaks may be associated with different weights.

According to an embodiment of the present invention, the synchronicity index may be based on a variability of the amplitude of detected peaks in systole and diastole, wherein increased amplitude variability corresponds to an increased value of the synchronicity index.

In an embodiment, the synchronicity index may be based on variability of the difference between peaks, for example, in systole and diastole, wherein increased variability corresponds to an increased value of the synchronicity index.

Moreover, in a further embodiment of the present invention, the synchronicity index is based on an absolute value of total peak amplitude of one or more of the detected peaks within the predetermined time window of in systole and diastole, wherein increased total peak amplitude corresponds to an increased value of the synchronicity index. In a further alternative, the synchronicity index may be the total area of the peaks in both systole and diastole, or a weighted value of all peaks where different peaks may be associated with different weights.

According to an embodiment of the present invention, a breath rate sensor is adapted to sense a breathing cycle of the patient, wherein the synchronicity index can be determined in synchronism with an event of a breathing cycle or respiration cycle of the patient or as an average value over a predetermined number of breathing cycles. Thereby, the accuracy of the synchronicity index can be significantly improved. This is due to the fact that the cardiogenic impedance is greatly affected by the respiration. Therefore, by synchronizing the determination of the synchronicity index with a particular event in the respiration cycle or by determining the index as an average value over a number of respiration cycles, the influence of the respiration on the impedance causing variability in the impedance signal can be eliminated or at least significantly reduced.

In a further embodiment of the present invention, a body posture sensor is adapted to sense a body posture of the patient, wherein the synchronicity index can be determined in synchronism with a predetermined body posture of the patient, or as an average value of the synchronicity index of at least two body postures. Thereby, the accuracy of the synchronicity index can be significantly improved. This is due to the fact that the cardiogenic impedance is greatly affected by the body posture of the patient. Therefore, by synchronizing the determination of the synchronicity index with a particular body posture or by determining the index as an average value over a more than one body posture, the influence of the body posture on the impedance causing variability in the impedance signal can be eliminated or at least significantly reduced.

Further embodiments include an activity sensor adapted to sense an activity level of the patient and a heart rate sensor adapted to sense a heart rate of the patient, respectively, and the synchronicity index may thus be determined in synchronism with a predetermined activity level of the patient or in synchronism with a predetermined heart rate or heart rate interval of the patient.

According to embodiments, one or several of the sensors including a heart rate sensor, a breath rate sensor, an activity sensor, and/or body posture sensor may be combined.

According to an embodiment, a matrix of synchronicity indices can be determined. For example, different indices for different body postures and for different activity levels may be included in the matrix. Further, different synchronicity indices may be determined for different events in the respiration cycle and for systole and diastole. During an optimization, the index corresponding to the present conditions of the patient can be selected and optimized.

In an embodiment of the present invention, IEGM signals of consecutive cardiac cycles are sensed and cardiac events are detected in the cardiac cycles using the IEGM signals. A time window is determined based on the detected cardiac events and the IEGM signals and/or impedance signals. A time window including a systolic phase of the cardiac cycle is determined to extend between a period starting at a detection of an R-wave and ending at a detection of a T-wave. Further, a time window including a diastolic phase of the cardiac cycle is determined to extend between a period starting at a detection of a T-wave and ending at a detection of a R-wave.

According to embodiments of the present invention, the impedance signal is measured using an electrode configuration including at least a first pair of electrodes having a ring electrode and a tip electrode arranged in a medical lead located in the right ventricle or a first electrode located adjacent to the septum in the right ventricle and a second electrode located in a coronary vein on the left ventricle or the can, i.e. the housing of the implantable medical device which may function as an electrode.

In an embodiment of the present invention, points of maximum amplitude of the impedance signal morphology are detected, wherein a first detected point at which the impedance reaches a maximum value is determined to be a first impedance peak and a second detected point at which the impedance reaches a maximum value is determined to be a second impedance peak. For example, points of the impedance amplitude where a first derivative of the impedance signal is zero and a second derivative of the impedance signal is below zero are determined to be impedance amplitude peaks.

According to the present invention, there is provided a system according to claim 9 for optimizing lead and/or electrode locations including an implantable medical device.

Hence, a test procedure is performed, for example, during an implantation of the implantable medical device according to the present invention so as to identify an optimal lead and/or electrode location with regard to inter alia capture and synchronized contraction of the ventricles. This may be performed by a physician using an external programmer unit connectable, via a cable or wirelessly, with the implantable medical device. During this procedure, for example, the optimal location of left ventricle electrodes can be determined. This can be achieved by starting with determined locations for a right ventricle lead and right ventricle electrodes and successively testing different locations for the left ventricle lead and/or left ventricle electrodes. At each test location, an optimization of a W interval can be performed to identify the minimum synchronicity index for that particular location. Thereafter, each synchronicity index (i.e. the index for each location) are compared to identify the overall minimum synchronicity index, which thus will correspond to the optimal location of the left ventricle lead and left ventricle electrode (-s). Of course, this procedure can also be performed to identify the optimal location for a right ventricular lead and right ventricular electrode (-s). Location of both left and/or right ventricular leads and electrodes can be optimized using the present invention. For example, a first location of the right ventricle lead and electrodes can be selected and a number of different left ventricle lead and electrode locations can be tested to identify the minimum synchronicity index. Then, a second location of the right ventricle lead and electrodes can be selected and all locations of the left ventricle lead are tested again to identify a minimum synchronicity index for this location. This is repeated for all possible locations of the right ventricle lead and electrodes. Consequently, a matrix of minimum synchronicity indices is obtained, and the overall minimum index can be selected, which will correspond to the optimal locations for left and right ventricular leads and electrodes.

Further objects and advantages of the present invention will be discussed below by means of exemplifying embodiments.

### Brief description of the drawings

Exemplifying embodiments of the invention will be described below with reference to the accompanying drawings, in which:
Fig. 1 schematically illustrates an implantable medical device according to an embodiment of the present invention;
Fig. 2 is schematic block diagram showing the implantable medical device of Fig. 1 in more detail;
Fig. 3a schematically shows cardiogenic impedance signal morphologies at the systolic phase during synchronous pacing/intrinsic functioning and asynchronous pacing/intrinsic functioning;
Fig. 3b schematically shows impedance signal morphologies at the diastolic phase during synchronous pacing/intrinsic functioning and asynchronous pacing/intrinsic functioning;
Fig. 4 shows measured cardiogenic impedance signals at biventricular and right ventricular pacing;
Fig. 5 shows the differentiated cardiogenic signal of Fig. 4 at biventricular and right ventricular pacing;
Fig. 6 is a flow chart showing the general principles for a method according to the present invention;
Fig. 7 is schematic diagram illustrating an embodiment of the present invention; and
Fig. 8 is a flow chart showing the general principles for an embodiment of the method according to the present invention.

### Description of exemplifying embodiments

The following is a description of exemplifying embodiments in accordance with the present invention. This description is not to be taken in limiting sense, but is made merely for the purposes of describing the general principles of the invention. Thus, even though particular types of implantable medical devices such as heart stimulators will be described, e.g. biventricular pacemakers, the invention is also applicable to other types of cardiac stimulators such as dual chamber stimulators, implantable cardioverter defibrillators (ICDs), etc.

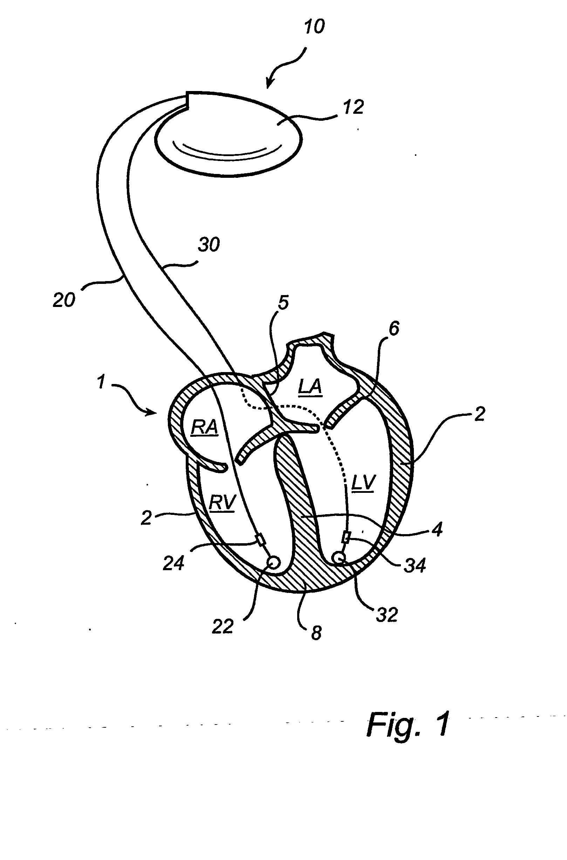

With reference first to Fig. 1, there is shown an implantable medical device according to an embodiment of the present invention. A stimulation device 10 is in electrical communication with a patient's heart 1 by way of medical leads 20 and 30 suitable for delivering multi-chamber stimulation, which leads 20 and 30 are connectable to the stimulator 10. The illustrated portions of the heart 1 include right atrium RA, the right ventricle RV, the left atrium LA, the left ventricle LV, cardiac walls 2, the ventricle septum 4, the valve plane 6, and the apex 8.

In order to sense right ventricular and atrial cardiac signals and impedances and to provide stimulation therapy to the right ventricle RV, the implantable medical device 10 is coupled to an implantable right ventricular lead 20, which may have a ventricular tip electrode 22 and a ventricular annular or ring electrode 24. The right ventricular tip electrode 22 is in this embodiment arranged to be implanted in the endocardium of the right ventricle, e.g. near the apex 8 of the heart. Thereby, the tip electrode 22 becomes attached to cardiac wall. In this example, the tip electrode 22 is fixedly mounted in a distal header portion of the lead 20.

Furthermore, in order to sense ventricular cardiac signals and impedances and to provide pacing therapy for the left ventricle LV, the implantable medical device 10 is coupled to a "coronary sinus" lead 30 designed for placement via the coronary sinus in veins located distally thereof, so as to place a distal electrode adjacent to the left ventricle. The coronary sinus lead 30 is designed to receive ventricular cardiac signals from the cardiac stimulator 10 and to deliver left ventricular LV pacing therapy using at least a left ventricular tip electrode 32 to the heart 1. In the illustrated example, the LV lead 30 further comprises an annular ring electrode 34 for sensing electrical activity related to the left ventricle LV of the heart.

With reference to the configuration shown in Fig. 1, a number of impedances vectors that can be used for obtaining impedance measurements reflecting the movements of the septal wall 4 will be described. For example, an impedance measurement wherein the current is applied between the ring electrode 24 of the right ventricle and the tip electrode 22 of the right ventricle, and the resulting impedance is measured between the same electrodes. A further alternative is an impedance measurement vector where the current is applied between the ring electrode 24 of the right ventricle and the case 12. The resulting impedance is measured between the same electrodes. As the skilled person realizes, there are a number of other conceivable measurement vectors that can be used to measure impedance reflecting the septal wall 4 movements, for example, between right ventricle electrodes located near the septal wall 4, 22 and/or 24 and left ventricle electrodes 32 and/or 34.

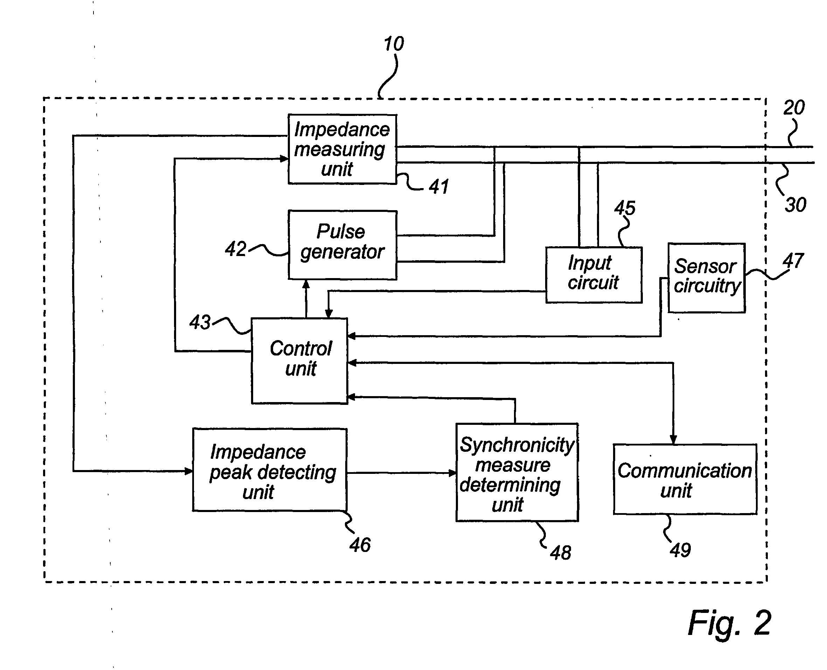

Turning now to Fig. 2, the heart stimulator 10 of Fig. 1 is shown in a block diagram form. For illustrative purposes, reference is made to Fig. 1 for the elements of the leads that are intended for positioning in or at the heart. an embodiment of the implantable medical device according to the present invention will be shown. The heart stimulator 10 comprises a housing 12 being hermetically sealed and biologically inert, see Fig. 1. Normally, the housing is conductive and may, thus, serve as an electrode. One or more pacemaker leads, where only two are shown in Fig. 1, 20 and 30, are electrically coupled to the implantable medical device 10 in a conventional manner. The leads 20, 30 extend into the heart (see Fig. 1) via a vein of the patient.

As discussed above with reference to Fig. 1, the leads 20, 30 comprises one or more electrodes, such a tip electrodes or a ring electrodes, arranged to, inter alia, transmit pacing pulses for causing depolarization of cardiac tissue adjacent to the electrode(-s) generated by a pace pulse generator 42 under influence of a control unit 43 comprising a microprocessor and for measuring impedances reflecting the septal wall movements. The control unit 43 controls, inter alia, pace pulse parameters such as output voltage and pulse duration. A memory circuit may be included in or connected to the control unit 43, which memory circuit may include a random access memory (RAM) and/or a non-volatile memory such as a read-only memory (ROM). Detected signals from the patients heart are processed in an input circuit 45 and are forwarded to the microprocessor of the control unit 43 for use in logic timing determination in known manner.

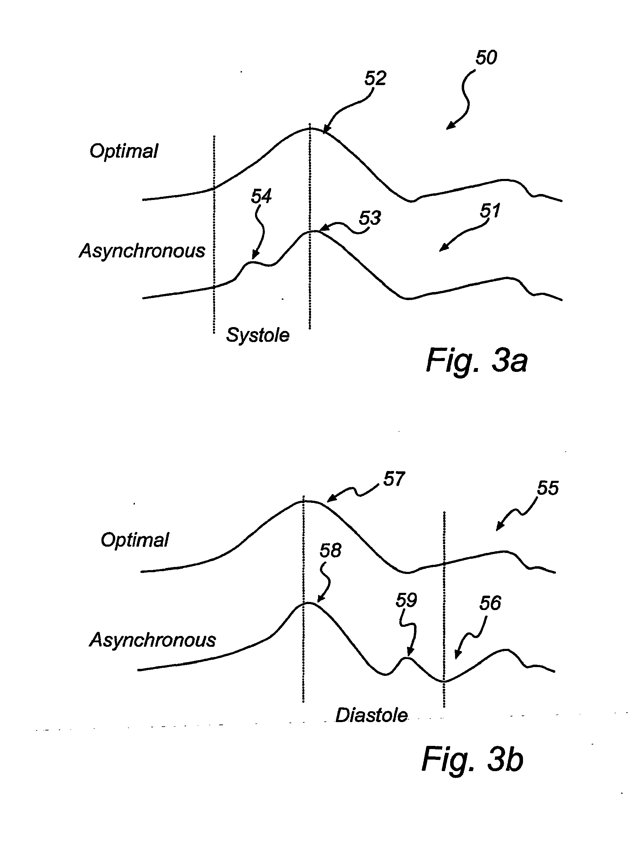

Furthermore, an impedance measuring unit 41 is adapted to carry out impedance measurements of the cardiac impedance of the patient indicative of the septal wall 4 movements, for example, by means of the measurements vectors wherein the current is applied between the ring electrode 24 of the right ventricle and the tip electrode 22 of the right ventricle, and the resulting impedance is measured between the same electrodes. A further alternative is an impedance measurement vector where the current is applied between the ring electrode 24 of the right ventricle lead and the tip electrode 32 of the left ventricle lead. The resulting impedance is measured between the same electrodes. Asynchronous ventricular contractions cause abnormal septal movements during systole as well as during diastole. The measured impedance reflects the mechanical motion of the septal wall 4 and, hence, it is possible to obtain a measure of the ventricular synchrony/asynchrony. This will be discussed in more detail below with reference to Figs. 3a and 3b.

The impedance measuring unit 41 may comprise an amplifier (not shown) that amplifies the evoked voltage response, i.e. the measured voltage, and may be synchronized with the excitation current. Thus, the impedance measuring unit 41 obtains the cardiac impedance given by the delivered current and the evoked voltage response. The impedance measuring unit 41 may also comprise a filtering circuit (not shown), for example, a Gaussian filter.

Furthermore, the heart stimulator 10 comprises an impedance peak detecting unit 46 adapted to determine an impedance signal morphology using measured impedance signals for consecutive cardiac cycles and to detect impedance amplitude peaks in the impedance signal morphology. The impedance peak detecting unit 46 thus identifies at least one characteristic shape feature of the impedance signal morphology being indicative of the occurrence of a synchronous/asynchronous depolarization of the left and right ventricles, respectively, i.e. amplitude peaks of the impedance. Asynchronous depolarization of the ventricles results in asynchronous myocardial contractions with regional dyskinetic cardiac tissue, which manifest in a peak split into two (or more) amplitude peaks in the impedance signal morphology.

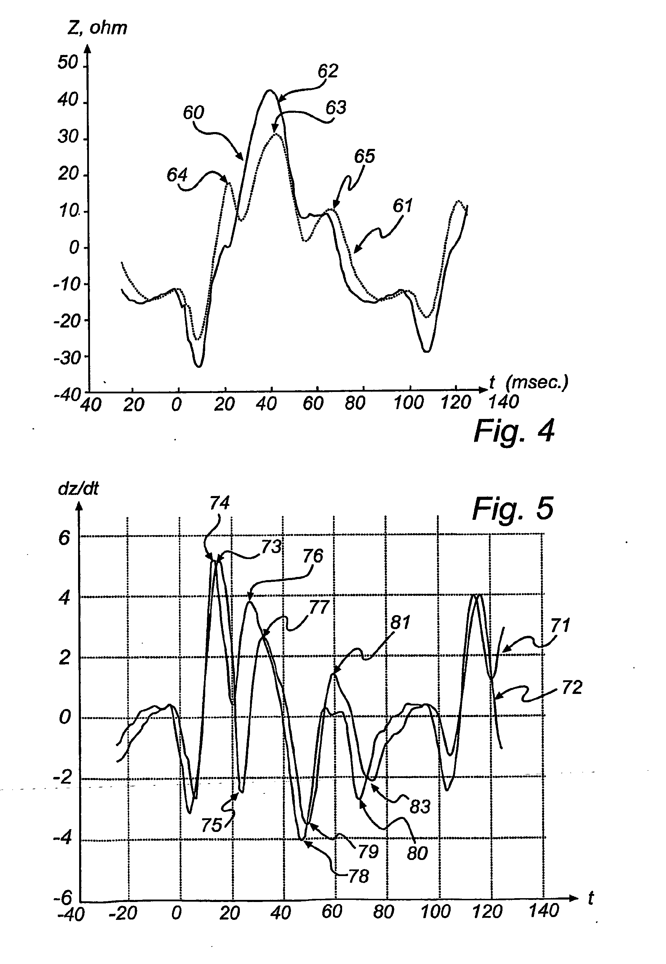

In one embodiment of the present invention, the impedance peak detecting unit 46 is adapted to differentiate the impedance signal to calculate a first derivative of the impedance signal and to detect points of local minima and/or local maxima of the first derivative as the impedance peaks (see Fig. 5, where diagrams of the derivative of the impedance signals obtained during biventricular pacing and during pacing in the right ventricular are displayed).

Further, the heart stimulator 10 further comprises a synchronicity index determining unit 48 adapted to determine a synchronicity index indicating a degree of synchronicity based on the detected impedance peaks. In one embodiment, at least two impedance peaks detected within a predetermined time window including a cardiac cycle or a part of a cardiac cycle correspond to an increased synchronicity index indicating an increased dyssynchronicity or asynchrony in the ventricular contractions.

The heart stimulator 10 also includes sensor circuits 47, for example, a respiration sensor for sensing a respiration rate or breathing rate and/or a body posture sensor for sensing a body posture of the patient. Additional sensors may include a heart rate sensor and/or an activity level sensor. The sensor circuit may be arranged in a medical lead 20, 30 or within the heart stimulator 10.

With reference now to Figs. 3a, 3b, 4 and 5, the impact on the impedance morphology resulting from asynchronous contractions will be discussed.

In Fig. 3a, impedance signal morphologies during the systolic phase are shown. In particular, the measured impedance during an optimal situation, i.e. at substantially synchronous ventricle contractions, indicated by the reference numeral 50, is shown in comparison with the measured impedance during ventricle asynchrony, indicated by the reference numeral 51. As can be seen, during the optimal situation, one pronounced main impedance amplitude peak 52 is present, whereas, during an asynchrony, two impedance peaks 53 and 54 have evolved. Still, during the asynchronous situation, one main impedance peak 53 can be identified but one minor impedance peak 54 has also evolved and can be identified in the impedance morphology. It can be noted that the minor impedance peak 54 occurs in relative close connection to the main peak 53, i.e. in close connection to the T-wave.

In Fig. 3b, impedance morphologies during the diastolic phase are shown. In particular, the measured impedance during an optimal situation, i.e. at synchronous ventricle contractions, indicated by the reference numeral 55, is shown in comparison with the measured impedance during ventricle asynchrony, which is indicated by the reference numeral 56. As can be seen, during the optimal situation one pronounced main impedance amplitude peak 57 is present. On the other hand, during the asynchrony, two impedance peaks 58 and 59 are present, one main peak 58 and one minor peak 59. It can be noted that the minor impedance peak 59 occurs in relative close connection to the main peak 58 or in close connection to the T-wave.

In Fig. 4, another situation is displayed where asynchronous contractions cause a split of the main impedance peak into three impedance peaks. An impedance signal measured during biventricular pacing (BiV), indicated by reference numeral 60, is compared with an impedance signal, indicated by reference numeral 61, measured during right ventricular pacing (RV). As can be seen, during biventricular pacing, one main impedance peak 62 can be identified in the impedance morphology. During the right ventricular pacing, two further impedance peaks 64 and 65 can be identified in addition to the main impedance peak 63. A first additional impedance peak 64 has evolved at the end of the systolic phase in close connection to the main peak 63 and a second additional impedance peak 65 has evolved in the beginning of the diastolic phase in close connection to the main peak 63.

In Fig. 5, the differentiated impedance of the impedance signals shown in Fig. 4 are displayed. The split waveform is shown clearly in the differentiated signals at systole and at diastole. The waveform 71 represents the differentiated impedance signal during biventricular stimulation and the waveform 72 represents the differentiated impedance signal during pacing in the right ventricular. In the differentiated signal 72 of the impedance signal obtained at right ventricular pacing, a number of local impedance maxima and minima 73, 77, 75, 79, and 83 can be identified, and in the differentiated signal 71 of the impedance signal obtained at bi-ventricular pacing a number of peaks, i.e. local maxima or minima, 74, 76, 78, and 80 can be identified.

According to an embodiment of the present invention, the synchronicity index is based on a peak distance between two detected peaks within the time window, wherein an increased peak distance corresponds to an increased value of the synchronicity index. With reference to Fig. 5, a synchronicity index may be based on the difference between detected peaks. For example, the difference between the local maxima 73 or the local maxima 77 and the local minima 75 is used as synchronicity index during systole. The higher difference between the respective peaks the higher synchronicity index. A higher synchronicity index indicates a higher degree of dyssynchronicity. In diastole, the difference between the maximum negative peak value 79 or 83 and the highest value 81 may be used as synchronicity index. During an optimization procedure, the W-interval that minimizes the synchronicity index is identified. Dependent on patient condition, the synchronization may be optimized during diastole, during systole or for both systole and diastole. For example, one synchronicity index may be determined for systole and one index for diastole and the optimization may be performed on basis of a weighted total index based on the two indices associated with different weights, i.e. the weighted total index is minimized. Alternatively, the indices may be optimized separately to obtain a minimized index in systole and in diastole, respectively.

According to another embodiment of the present invention, the synchronicity index is based on the number of detected peaks within the predetermined time window, wherein an increased number of peaks corresponds to an increased value of the synchronicity index. With reference to Fig. 5, the peaks 73, 75, and 77 may constitute the synchronicity index at systole, and the peaks 79, 81, and 83 may constitute the index at diastole. Alternatively, the all peaks 73, 75, 77, 79, 81, and 83 may constitute the synchronicity index.

In a further embodiment of the present invention, the synchronicity index is based on a total peak area of one or several detected peaks within the predetermined time window measured above a predetermined threshold, wherein an increased peak area corresponds to an increased value of the synchronicity index. Referring to Fig. 5, the synchronicity index for systole may be the area of the peak 73 or 77 above a predetermined level or threshold or the area of the peak 75 below a predetermined level or threshold. The area may be determined by integrating the peaks 73 and/or 77 above the level or threshold or by integrating the peak 75 below the level or threshold. Alternatively, the synchronicity index may be the total area of the peaks 73, 75 and 77 in systole. At diastole, the synchronicity index may be the area of the peak 81 above a predetermined level or threshold or the area of the peaks 79 or 83 below a predetermined level or threshold. Alternatively, the synchronicity index may be the total area of the peaks 79, 81, and 83 in diastole. In a further alternative, the synchronicity index may be the total area of the peaks in both systole and diastole, or a weighted value of all peaks where different peaks may be associated with different weights.

According to another embodiment of the present invention, the synchronicity index is based on a variability of the amplitude of detected peaks, wherein increased amplitude variability corresponds to an increased value of the synchronicity index. Further, the variability of the difference between peaks, for example, between the local maximum peaks 73 or 77 and the local minimum peak 75 shown in Fig. 5 can be used as the synchronicity index.

Moreover, in a further embodiment of the present invention, the synchronicity index is based on an absolute value of total peak amplitude of the detected peaks within the predetermined time window, wherein increased total peak amplitude corresponds to an increased value of the synchronicity index. Referring to Fig. 5, the synchronicity index for systole may be the amplitude of the peak 73 or 77 or the amplitude of the peak 75. Alternatively, the synchronicity index may be the total amplitude of the peaks 73, 75 and 77 in systole. At diastole, the synchronicity index may be the total amplitude of the peak 81 or the amplitude of the peaks 79 or 83. Alternatively, the synchronicity index may be the total amplitude of the peaks 79, 81, and 83 in diastole. In a further alternative, the synchronicity index may be the total area of the peaks in both systole and diastole, or a weighted value of all peaks where different peaks may be associated with different weights.

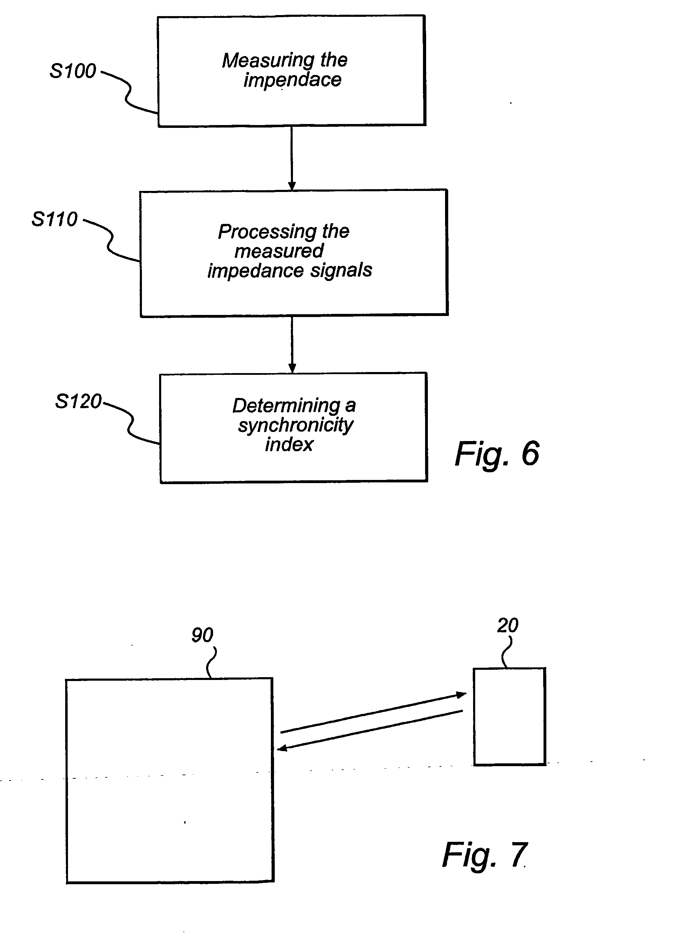

With reference now to Fig. 6, the general concept of monitoring ventricular synchrony of a heart according to the present invention will be described. The method may be implemented in an implantable medical device (e.g. a device described above with reference to Fig. 1 and 2) comprising a pace pulse generator adapted to produce cardiac stimulating pacing pulses and being connectable to at least one medical lead for delivering stimulation pulses to cardiac tissue of the heart. The method includes a first step, S100, of, during impedance measuring sessions, measuring impedance signals by an electrode configuration being located such that the impedance signals substantially reflects septal wall movements, wherein the electrodes of the electrode configuration are connectable to the implantable medical device. The, at step S110, the impedance signals are processed to determine an impedance signal morphology and to detect impedance amplitude peaks in the impedance signal morphology. Thereafter, at step S120, a synchronicity index indicating a degree of synchronicity is determined based on detected impedance peaks, wherein at least two impedance peaks detected within a predetermined time window including a cardiac cycle or a part of a cardiac cycle indicates an increased dyssynchronicity in the ventricular contractions.

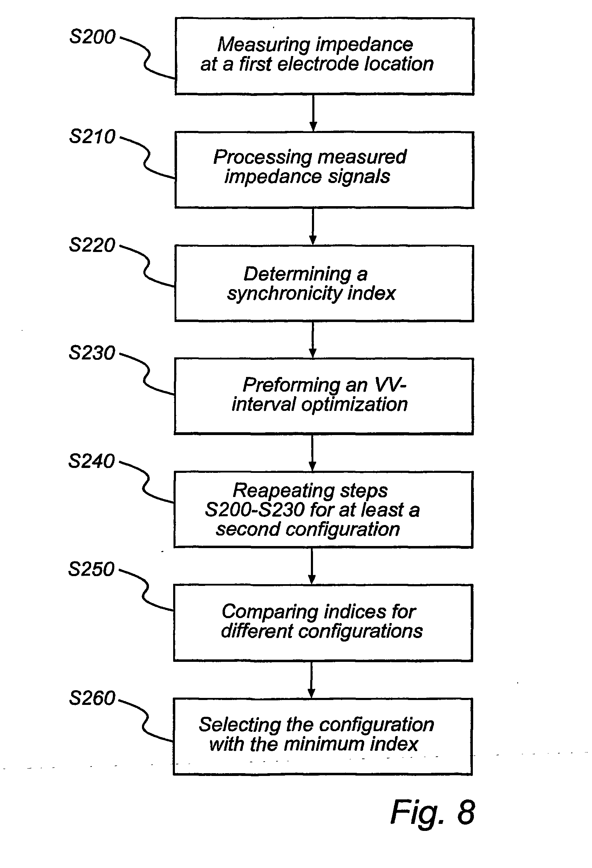

According to another aspect of the present invention, an optimization procedure so as to find or identify the optimal lead and/or electrode location can be performed. For example, during an implantation of an implantable medical device according to the present invention, a physician can perform such an optimization. In such a case, an external programmer unit 90, with reference to Fig. 7, can be connected, e.g. wirelessly or via cable, to the implantable medical device 10 to allow the physician to monitor and perform the optimization procedure. The bi-directional transition of information between the programmer unit 90 and the implantable medical device 10 can be executed, for example, by means of telemetry or RF via the communication unit 49 of the implantable medical device 10. Referring to Fig. 8, such a method for optimizing lead and/or electrode locations will be briefly discussed. First, at step S200, impedance signals at a first electrode configuration located such that the impedance signals substantially reflects septal wall movements is measured, wherein the electrodes of the electrode configuration are connectable to the implantable medical device and are located at a right side and/or left side of the heart. Then, at step 210, the impedance signals are processed to determine impedance signal morphology and to detect impedance amplitude peaks in the impedance signal morphology. At step S220, a synchronicity index indicating a degree of synchronicity is determined based on detected impedance peaks for the first electrode configuration, wherein at least two impedance peaks detected within a predetermined time window including a cardiac cycle or a part of a cardiac cycle indicates an increased dyssynchronicity in the ventricular contractions. Thereafter, at step S230, an optimization procedure is performed based on the synchronicity index by iteratively adjust a W-interval so as to minimize the synchronicity index in the predetermined time window for the first electrode configuration. At step S240, steps S200-S230 are repeated for at least a second electrode configuration. Preferably, this is repeated for all possible or all desired electrode configurations. For example, the optimal location of left ventricle electrodes can be determined. This can be achieved by starting with determined locations for a right ventricle lead and right ventricle electrodes and successively testing different locations for the left ventricle lead and left ventricle electrodes. At each test location, an optimization of a W interval can be performed to identify the minimum synchronicity index for that particular location. Thereafter, each synchronicity index (i.e. the index for each location) are compared to identify the overall minimum synchronicity index, which thus will correspond to the optimal location of the left ventricle lead and left ventricle electrode (-s). Of course, this procedure can also be performed to identify the optimal location for a right ventricular lead and right ventricular electrode (-s). Both left and right ventricular leads and electrodes can be optimized using the present invention. For example, a first location of the right ventricle lead and electrodes can be selected and a number of different left ventricle lead and electrode locations can be tested to identify the minimum synchronicity index. Then, a second location of the right ventricle lead and electrodes can be selected and all locations of the left ventricle lead are tested again to identify a minimum synchronicity index for this location. This is repeated for all possible locations of the right ventricle lead and electrodes. Consequently, a matrix of minimum synchronicity indices is obtained, and the overall minimum index can be selected, which will correspond to the optimal locations for left and right ventricular leads and electrodes. However, the method according to this further aspect may also be used within an implanted medical device to optimize an electrode configuration if the leads comprise a number of possible electrode configurations. When all possible locations or all desired locations have been tested, at step S250, the minimum synchronicity index for each configuration is compared to identify an overall minimum synchronicity index. Then, at step S260, the electrode configuration being associated with the minimum synchronicity index is selected as the optimal electrode configuration or the optimal lead location.

According to yet another embodiment of the present invention, a pacing analyzer according to claim 11 for optimizing lead and/or electrode locations is connectable to at least one medical lead implantable in a heart of a patient. A pacing analyzer is used to assess the electrical performance of a lead system during implantation of a heart stimulator, e.g. a stimulator as described above with reference to Figs. 1 and 2, or invasive lead-system trouble shooting.

Thus, a physician can use the pacing analyzer to optimize lead and/or electrode locations during, for example, implantation. First, the pacing analyzer is connected to the medical lead or leads. Then, impedance signals at a first electrode configuration located such that the impedance signals substantially reflects septal wall movements is measured, wherein the electrodes of the electrode configuration are connectable to the implantable medical device and are located at a right side and/or left side of the heart. Then, the impedance signals are processed to determine impedance signal morphology and to detect impedance amplitude peaks in the impedance signal morphology. A synchronicity index indicating a degree of synchronicity is determined based on detected impedance peaks for the first electrode configuration, wherein at least two impedance peaks detected within a predetermined time window including a cardiac cycle or a part of a cardiac cycle indicates an increased dyssynchronicity in the ventricular contractions. Thereafter, an optimization procedure is performed based on the synchronicity index by iteratively adjust a W-interval so as to minimize the synchronicity index in the predetermined time window for the first electrode configuration. Further, the preceding steps are repeated for at least a second electrode configuration. Preferably, this is repeated for all possible or all desired electrode configurations. For example, the optimal location of left ventricle electrodes can be determined. This can be achieved by starting with determined locations for a right ventricle lead and right ventricle electrodes and successively testing different locations for the left ventricle lead and left ventricle electrodes. At each test location, an optimization of a W interval can be performed to identify the minimum synchronicity index for that particular location. Thereafter, each synchronicity index (i.e. the index for each location) are compared to identify the overall minimum synchronicity index, which thus will correspond to the optimal location of the left ventricle lead and left ventricle electrode (-s). Of course, this procedure can also be performed to identify the optimal location for a right ventricular lead and right ventricular electrode (-s). Both left and right ventricular leads and electrodes can be optimized using the present invention. For example, a first location of the right ventricle lead and electrodes can be selected and a number of different left ventricle lead and electrode locations can be tested to identify the minimum synchronicity index. Then, a second location of the right ventricle lead and electrodes can be selected and all locations of the left ventricle lead are tested again to identify a minimum synchronicity index for this location. This is repeated for all possible locations of the right ventricle lead and electrodes. Consequently, a matrix of minimum synchronicity indices is obtained, and the overall minimum index can be selected, which will correspond to the optimal locations for left and right ventricular leads and electrodes, However, the method may also be used within an implanted medical device to optimize an electrode configuration if the leads comprise a number of possible electrode configurations. When all possible locations or all desired locations have been tested, the minimum synchronicity index for each configuration is compared to identify an overall minimum synchronicity index. Then, the electrode configuration being associated with the minimum synchronicity index is selected as the optimal electrode configuration or the optimal lead location.

Although exemplary embodiments of the present invention has been shown and described, it will be apparent to those having ordinary skill in the art that a number of changes, modifications, or alterations to the inventions as described herein may be made. Thus, it is to be understood that the above description of the invention and the accompanying drawings is to be regarded as a non-limiting example thereof and that the scope of protection is defined by the appended patent claims.

## Claims

1. An implantable medical device (10) for monitoring ventricular synchrony of a heart including a pace pulse generator (42) adapted to produce cardiac stimulating pacing pulses and being connectable to at least one medical lead (20,30) for delivering stimulation pulses to cardiac tissue of said heart, comprising:
an impedance measuring unit (41) adapted to, during impedance measuring sessions, measure impedance signals obtained by an electrode configuration being located such that said impedance signals substantially reflects septal wall movements, wherein the electrodes of said electrode configuration are connectable to said implantable medical device;
an impedance peak detecting unit (46) adapted to process said impedance signals to determine an impedance signal morphology and to detect impedance amplitude peaks in said impedance signal morphology; and **characterized by**:
a synchronicity index determining unit (48) adapted to:
determine a first synchronicity index indicating a degree of synchronicity based on detected impedance peaks within a time window corresponding to systole, wherein at least two impedance peaks indicates an increased dyssynchronicity;
determine a second synchronicity index indicating a degree of synchronicity based on detected impedance peaks within a time window corresponding to diastole, wherein at least two impedance peaks indicates an increased dyssynchronicity;
associate a weight to said first and second synchronicity index, respectively; and
optimize a total synchronicity index based on said first and second synchronicity index to obtain a minimized total index or optimizing said first and second synchronicity index separately to obtain a minimized index in systole and diastole, respectively.

2. The implantable medical device according to any one of preceding claims, said impedance peak detecting unit is adapted to calculate a first derivative of said impedance signal and to detect points of local minima and/or local maxima of said first derivative as impedance peaks.

3. The implantable medical device according to claim 1 or 2, wherein said synchronicity index determining unit is adapted to determine a synchronicity index based on:
- a peak distance between two detected peaks within said time window, wherein an increased peak distance corresponds to an increased value of the synchronicity index, and/ or
- a number of detected peaks within said predetermined time window, wherein an increased number of peaks corresponds to an increased value of the synchronicity index, and/or
- a total peak area of detected peaks within said predetermined time window measured above a predetermined threshold, wherein an increased peak area corresponds to an increased value of the synchronicity index; and/or
- a variability of the amplitude of detected peaks, wherein an increased amplitude variability corresponds to an increased value of the synchronicity index; and/or
- an absolute value of a total peak amplitude of the detected peaks within said predetermined time window, wherein an increased total peak amplitude corresponds to an increased value of the synchronicity index.

4. The implantable medical device according to any one of preceding claims, further including a breath rate sensor adapted to sense a breathing cycle of said patient, wherein said synchronicity determining unit is adapted to determine said synchronicity index in synchronism with an event of said a breathing cycle of said patient or as an average value over a predetermined number of breathing cycles.

5. The implantable medical device according to any one of preceding claims, further comprising a body posture sensor adapted to sense a body posture of said patient, wherein said synchronicity determining unit is adapted to determine said synchronicity index in synchronism with a predetermined body posture of said patient, or as an average value of the synchronicity index of at least two body postures.

6. The implantable medical device according to any one of preceding claims, further comprising a W delay determining unit adapted to perform an optimization procedure, wherein said pace pulse generator is controlled to, based on said synchronicity index, iteratively adjust a W-interval so as to minimize said synchronicity index in said predetermined time window to obtain substantially synchronized ventricle contractions.

7. The implantable medical device according to any one of preceding claims, further comprising a IEGM circuit adapted to sense IEGM signals of consecutive cardiac cycles and to detect cardiac events in said cardiac cycles using said IEGM signals; and
wherein said synchronicity measure determining circuit is adapted to determine predetermined time window based on said detected cardiac events and said IEGM signals and/or impedance signals.

8. The implantable medical device according to anyone of preceding claims, wherein said electrode configuration includes at least a first pair of electrodes having a ring electrode and a tip electrode arranged in a medical lead located in the right ventricle, or a first electrode located adjacent to the septum in the right ventricle and a second electrode located in a coronary vein on the left ventricle or the can.

9. A system for optimizing lead and/or electrode locations including an implantable medical device (10), said device including
a pace pulse generator (42) adapted to produce cardiac stimulating pacing pulses and being connectable to at least one medical lead for delivering stimulation pulses to cardiac tissue of said heart;
an impedance measuring unit (41) adapted to, during impedance measuring sessions, measure impedance signals obtained at a first electrode configuration being located such that said impedance signals substantially reflects septal wall movements, wherein the electrodes of said electrode configuration are connectable to said device;
an impedance peak detecting unit (46) adapted to process said impedance signals to determine an impedance signal morphology and to detect impedance amplitude peaks in said impedance signal morphology;
a synchronicity index determining unit (48) adapted to determine a synchronicity index indicating a degree of synchronicity based on detected impedance peaks for said first electrode configuration, **characterised in that**:
said synchronicity index determining unit is adapted to:
determine a first synchronicity index indicating a degree of synchronicity based on detected impedance peaks within a time window corresponding to systole, wherein at least two impedance peaks indicates an increased dyssynchronicity;
determine a second synchronicity index indicating a degree of synchronicity based on detected impedance peaks within a time window corresponding to diastole, wherein at least two impedance peaks indicates an increased dyssynchronicity;
associate a weight to said first and second synchronicity index, respectively; and
optimize a total synchronicity index based on said first and second synchronicity index to obtain a minimized total index or optimizing said first and second synchronicity index separately to obtain a minimized index in systole and diastole, respectively
a W delay determining unit adapted to perform an optimization procedure, wherein said pace pulse generator is controlled to, based on said synchronicity index, iteratively adjust a W-interval so as to minimize said synchronicity index in said predetermined time window; and
an external control unit (90) connectable to said implantable medical device and being adapted to:
instruct said implantable medical device to obtain a synchronicity index for at least a second electrode configuration;
compare said minimum synchronicity index for each configuration to identify a overall minimum synchronicity index; and
select the electrode configuration being associated with the minimum synchronicity index.

10. The system according to claim 9, wherein said implantable medical device is adapted in accordance with any one of preceding claims 2-5, or 7.

11. An pacing analyzer for optimizing lead and/or electrode locations being connectable to at least one medical lead implantable in a heart of a patient, said analyzer including
a pace pulse generator adapted to produce cardiac stimulating pacing pulses and being connectable to at least one medical lead for delivering stimulation pulses to cardiac tissue of said heart;
an impedance measuring unit adapted to, during impedance measuring sessions, measure impedance signals obtained at an electrode configuration and/or lead configuration being located such that said impedance signals substantially reflects septal wall movements, wherein the electrodes of said electrode configuration are connectable to said device;
an impedance peak detecting unit adapted to process said impedance signals to determine an impedance signal morphology and to detect impedance amplitude peaks in said impedance signal morphology;
a synchronicity index determining unit adapted to determine a synchronicity index indicating a degree of synchronicity based on detected impedance peaks for said electrode configuration, **characterised in that** said synchronicity index determining unit is adapted to:
determine a first synchronicity index indicating a degree of synchronicity based on detected impedance peaks within a time window corresponding to systole, wherein at least two impedance peaks indicates an increased dyssynchronicity:
determine a second synchronicity index indicating a degree of synchronicity based on detected impedance peaks within a time window corresponding to diastole, wherein at least two impedance indicates an increased dyssynchronicity;
associate a weight to said first and second synchronicity index, respectively: and
optimize a total synchronicity index based on said first and second synchronicity index to obtain a minimized total index or optimizing said first and second synchronicity index separately to obtain a minimized index in systole and diastole, respectively;
a W delay determining unit adapted to perform an optimization procedure, wherein said pace pulse generator is controlled to, based on said synchronicity index, iteratively adjust a W-interval so as to minimize said synchronicity index in said predetermined time window; and
a control unit adapted to:
compare said minimum synchronicity index for different electrode and/or lead configurations to identify a overall minimum synchronicity index; and
indicate the electrode configuration being associated with the minimum synchronicity index.

## Patentansprüche

1. Implantierbares Medizinprodukt (10) zum Überwachen einer Herzkammersynchronität eines Herzens mit einem Stimulierungsimpulsgenerator (42), der so ausgelegt ist, dass er Herzstimulationserregungsimpulse erzeugt und mit mindestens einer medizinischen Leitung (20, 30) zum Abgeben von Stimulationsimpulsen an Herzgewebe des Herzens verbindbar ist, umfassend:
eine Impedanzmesseinheit (41), die so ausgelegt ist, dass sie während Impedanzmessdurchläufen Impedanzsignale misst, die von einer Elektrodenanordnung erhalten werden, die so angeordnet ist, dass die Impedanzsignale im Wesentlichen Scheidewandbewegungen abbilden, wobei die Elektroden der Elektrodenanordnung mit dem implantierbaren Medizinprodukt verbindbar sind;
eine Impedanzspitzen-Erkennungseinheit (46), die so ausgelegt ist, dass sie die Impedanzsignale verarbeitet, damit sie eine Impedanzsignalmorphologie bestimmt und Impedanzamplitudenspitzen in der Impedanzsignalmorphologie erkennt; und
**gekennzeichnet durch:**
eine Synchronizitätsindexbestimmungseinheit (48), die so ausgelegt ist, dass sie:
einen ersten Synchronizitätsindex, der einen Synchronizitätsgrad kennzeichnet, auf der Grundlage von erkannten Impedanzspitzen innerhalb eines Zeitfensters bestimmt, das einer Systole entspricht, wobei mindestens zwei Impedanzspitzen eine erhöhte Dyssynchronizität kennzeichnen;
einen zweiten Synchronizitätsindex, der einen Synchronizitätsgrad kennzeichnet, auf der Grundlage von erkannten Impedanzspitzen innerhalb eines Zeitfensters bestimmt, das einer Diastole entspricht, wobei mindestens zwei Impedanzspitzen eine erhöhte Dyssynchronizität kennzeichnen;
dem ersten beziehungsweise zweiten Synchronizitätsindex ein Gewicht zuordnet; und
einen Gesamtsynchronizitätsindex auf der Grundlage des ersten Synchronizitätsindex und zweiten Synchronizitätsindex optimiert, damit sie einen minimierten Gesamtindex ermittelt, oder den ersten und zweiten Synchronizitätsindex separat optimiert, damit sie einen minimierten Index in der Systole beziehungsweise Diastole ermittelt.

2. Implantierbares Medizinprodukt nach einem der vorhergehenden Ansprüche, wobei die Impedanzspitzen-Erkennungseinheit so ausgelegt ist, dass sie eine erste Ableitung des Impedanzsignals berechnet und lokale Minimal- und/oder lokale Maximalstellen der ersten Ableitung als Impedanzspitzen erkennt.

3. Implantierbares Medizinprodukt nach Anspruch 1 oder 2, wobei die Synchronizitätsindexbestimmungseinheit so ausgelegt ist, dass sie einen Synchronizitätsindex bestimmt auf der Grundlage von:
- einem Spitzenabstand zwischen zwei erkannten Spitzen innerhalb des Zeitfensters, wobei ein vergrößerter Spitzenabstand einem erhöhten Wert des Synchronizitätsindex entspricht, und/ oder
- einer Anzahl von erkannten Spitzen in dem vorher festgelegten Zeitfenster, wobei eine erhöhte Anzahl von Spitzen einem erhöhten Wert des Synchronizitätsindex entspricht, und/ oder
- einer Gesamtspitzenfläche erkannter Spitzen in dem vorher festgelegten Zeitfenster, gemessen über einem vorher festgelegten Schwellwert, wobei eine erhöhte Spitzenfläche einem erhöhten Wert des Synchronizitätsindex entspricht; und/oder
- einer Variabilität der Amplitude erkannter Spitzen, wobei eine erhöhte Amplitudenvariabilität einem erhöhten Wert des Synchronizitätsindex entspricht; und/oder
- einem Absolutwert einer Gesamtspitzenamplitude der erkannten Spitzen in dem vorher festgelegten Zeitfenster, wobei eine erhöhte Gesamtspitzenamplitude einem erhöhten Wert des Synchronizitätsindex entspricht.

4. Implantierbares Medizinprodukt nach einem der vorhergehenden Ansprüche, das ferner einen Atemfrequenzsensor aufweist, der so ausgelegt ist, dass er einen Atemzyklus des Patienten erfasst, wobei die Synchronizitätsbestimmungseinheit so ausgelegt ist, dass sie den Synchronizitätsindex synchron mit einem Ereignis des Atemzyklus des Patienten oder als Durchschnittswert über eine vorher festgelegte Anzahl von Atemzyklen bestimmt.

5. Implantierbares Medizinprodukt nach einem der vorhergehenden Ansprüche, das ferner einen Körperhaltungssensor umfasst, der so ausgelegt ist, dass er eine Körperhaltung des Patienten erfasst, wobei die Synchronizitätsbestimmungseinheit so ausgelegt ist, dass sie den Synchronizitätsindex synchron mit einer vorher festgelegten Körperhaltung des Patienten oder als Durchschnittswert des Synchronizitätsindex aus mindestens zwei Körperhaltungen bestimmt.

6. Implantierbares Medizinprodukt nach einem der vorhergehenden Ansprüche, das ferner eine W-Intervall-Bestimmungseinheit umfasst, die so ausgelegt ist, dass sie einen Optimierungsvorgang vornimmt, wobei der Stimulierungsimpulsgenerator so gesteuert ist, dass er auf der Grundlage des Synchronizitätsindex ein W-Intervall iterativ anpasst, damit der Synchronizitätsindex in dem vorher festgelegten Zeitfenster minimiert wird und so im Wesentlichen synchronisierte Herzkammerkontraktionen erhalten werden.

7. Implantierbares Medizinprodukt nach einem der vorhergehenden Ansprüche, das ferner eine IEGM-Schaltung umfasst, die so ausgelegt ist, dass sie IEGM-Signale aufeinanderfolgender Herzzyklen erfasst und Herzereignisse in den Herzzyklen unter Verwendung der IEGM-Signale erkennt; und
wobei die Schaltung zur Bestimmung des Synchronizitätsmaßes so ausgelegt ist, dass sie das vorher festgelegte Zeitfenster auf der Grundlage der erkannten Herzereignisse und der IEGM-Signale und/oder Impedanzsignale festlegt.

8. Implantierbares Medizinprodukt nach einem der vorhergehenden Ansprüche, wobei die Elektrodenanordnung mindestens ein erstes Paar von Elektroden mit einer Ringelektrode und einer Spitzenelektrode, die in einer medizinischen Leitung angeordnet sind, die sich in der rechten Herzkammer befindet, oder eine erste Elektrode, die sich angrenzend an die Scheidewand in der rechten Herzkammer befindet, und eine zweite Elektrode, die sich in einer Koronarvene an der linken Herzkammer befindet, oder das Gehäuse aufweist.

9. System zum Optimieren der Leitungs- und/oder Elektrodenlage mit einem implantierbaren Medizinprodukt (10), wobei das Produkt Folgendes aufweist:
einen Stimulierungsimpulsgenerator (42), der so ausgelegt ist, dass er Herzstimulationserregungsimpulse erzeugt und mit mindestens einer medizinischen Leitung zum Abgeben von Stimulationsimpulsen an Herzgewebe des Herzens verbindbar ist;
eine Impedanzmesseinheit (41), die so ausgelegt ist, dass sie während Impedanzmessdurchläufen Impedanzsignale misst, die an einer ersten Elektrodenanordnung erhalten werden, die so angeordnet ist, dass die Impedanzsignale im Wesentlichen Scheidewandbewegungen abbilden, wobei die Elektroden der Elektrodenanordnung mit dem Produkt verbindbar sind;
eine Impedanzspitzen-Erkennungseinheit (46), die so ausgelegt ist, dass sie die Impedanzsignale verarbeitet, damit sie eine Impedanzsignalmorphologie bestimmt und Impedanzamplitudenspitzen in der Impedanzsignalmorphologie erkennt;
eine Synchronizitätsindexbestimmungseinheit (48), die so ausgelegt ist, dass sie einen Synchronizitätsindex, der einen Synchronizitätsgrad kennzeichnet, auf der Grundlage von erkannten Impedanzspitzen für die erste Elektrodenanordnung bestimmt,
**dadurch gekennzeichnet, dass:**
die Synchronizitätsindexbestimmungseinheit so ausgelegt ist, dass sie:
einen ersten Synchronizitätsindex, der einen Synchronizitätsgrad kennzeichnet, auf der Grundlage von erkannten Impedanzspitzen innerhalb eines Zeitfensters bestimmt, das einer Systole entspricht, wobei mindestens zwei Impedanzspitzen eine erhöhte Dyssynchronizität kennzeichnen;
einen zweiten Synchronizitätsindex, der einen Synchronizitätsgrad kennzeichnet, auf der Grundlage von erkannten Impedanzspitzen innerhalb eines Zeitfensters bestimmt, das einer Diastole entspricht, wobei mindestens zwei Impedanzspitzen eine erhöhte Dyssynchronizität kennzeichnen;
dem ersten beziehungsweise zweiten Synchronizitätsindex ein Gewicht zuordnet; und
einen Gesamtsynchronizitätsindex auf der Grundlage des ersten und zweiten Synchronizitätsindex optimiert, damit sie einen minimierten Gesamtindex ermittelt, oder den ersten und zweiten Synchronizitätsindex separat optimiert, damit sie einen minimierten Index in der Systole beziehungsweise Diastole ermittelt
eine W-Intervall-Bestimmungseinheit, die so ausgelegt ist, dass sie einen Optimierungsvorgang vornimmt, wobei der Stimulierungsimpulsgenerator so gesteuert ist, dass er auf der Grundlage des Synchronizitätsindex ein W-Intervall iterativ anpasst, damit der Synchronizitätsindex in dem vorher festgelegten Zeitfenster minimiert wird; und
eine externe Steuereinheit (90), die mit dem implantierbaren Medizinprodukt verbindbar und so ausgelegt ist, dass sie:
das implantierbare Medizinprodukt dazu anweist, einen Synchronizitätsindex für mindestens eine zweite Elektrodenanordnung zu ermitteln;
das Synchronizitätsindexminimum für jede Anordnung vergleicht und so ein Gesamtsynchronizitätsindexminimum ermittelt; und
die Elektrodenanordnung auswählt, die dem Synchronizitätsindexminimum zugehörig ist.

10. System nach Anspruch 9, wobei das implantierbare Medizinprodukt nach einem der vorhergehenden Ansprüche 2 bis 5 oder Anspruch 7 ausgelegt ist.

11. Erregungsanalysator zum Optimieren der Leitungs- und/oder Elektrodenlage, der mit mindestens einer medizinischen Leitung verbindbar ist, die in einem Herz eines Patienten implantierbar ist, wobei der Analysator Folgendes aufweist:
einen Stimulierungsimpulsgenerator, der so ausgelegt ist, dass er Herzstimulationserregungsimpulse erzeugt und mit mindestens einer medizinischen Leitung zum Abgeben von Stimulationsimpulsen an Herzgewebe des Herzens verbindbar ist;
eine Impedanzmesseinheit, die so ausgelegt ist, dass sie während Impedanzmessdurchläufen Impedanzsignale misst, die an einer Elektrodenanordnung und/oder Leitungsanordnung erhalten werden, die so angeordnet ist bzw. sind, dass die Impedanzsignale im Wesentlichen Scheidewandbewegungen abbilden, wobei die Elektroden der Elektrodenanordnung mit dem Produkt verbindbar sind;
eine Impedanzspitzen-Erkennungseinheit, die so ausgelegt ist, dass sie die Impedanzsignale verarbeitet, damit sie eine Impedanzsignalmorphologie bestimmt und Impedanzamplitudenspitzen in der Impedanzsignalmorphologie erkennt;
eine Synchronizitätsindexbestimmungseinheit, die so ausgelegt ist, dass sie einen Synchronizitätsindex, der einen Synchronizitätsgrad kennzeichnet, auf der Grundlage von erkannten Impedanzspitzen für die Elektrodenanordnung bestimmt,
**dadurch gekennzeichnet, dass:**
die Synchronizitätsindexbestimmungseinheit so ausgelegt ist, dass sie:
einen ersten Synchronizitätsindex, der einen Synchronizitätsgrad kennzeichnet, auf der Grundlage von erkannten Impedanzspitzen innerhalb eines Zeitfensters bestimmt, das einer Systole entspricht, wobei mindestens zwei Impedanzspitzen eine erhöhte Dyssynchronizität kennzeichnen;
einen zweiten Synchronizitätsindex, der einen Synchronizitätsgrad kennzeichnet, auf der Grundlage von erkannten Impedanzspitzen innerhalb eines Zeitfensters bestimmt, das einer Diastole entspricht, wobei mindestens zwei Impedanzspitzen eine erhöhte Dyssynchronizität kennzeichnen;
dem ersten beziehungsweise zweiten Synchronizitätsindex ein Gewicht zuordnet; und
einen Gesamtsynchronizitätsindex auf der Grundlage des ersten und zweiten Synchronizitätsindex optimiert, damit sie einen minimierten Gesamtindex ermittelt, oder den ersten und zweiten Synchronizitätsindex separat optimiert, damit sie einen minimierten Index in der Systole beziehungsweise Diastole ermittelt;
eine W-Intervall-Bestimmungseinheit, die so ausgelegt ist, dass sie einen Optimierungsvorgang vornimmt, wobei der Stimulierungsimpulsgenerator so gesteuert ist, dass er auf der Grundlage des Synchronizitätsindex ein W-Intervall iterativ anpasst, damit der Synchronizitätsindex in dem vorher festgelegten Zeitfenster minimiert wird; und
eine Steuereinheit, die so ausgelegt ist, dass sie:
das Synchronizitätsindexminimum für verschiedene Elektroden- und/oder Leitungsanordnungen vergleicht und so ein Gesamtsynchronizitätsindexminimum ermittelt; und
die Elektrodenanordnung angibt, die dem
Synchronizitätsindexminimum zugehörig ist.

## Revendications

1. Dispositif médical implantable (10) de surveillance de synchronicité ventriculaire d'un coeur comportant un générateur d'impulsions de stimulation (42) adapté pour produire des impulsions régulatrices de stimulation cardiaque et qui peut être connecté à au moins une dérivation médicale (20, 30) pour transmettre des impulsions de stimulation à du tissu cardiaque dudit coeur, comprenant :
une unité de mesure d'impédance (41) adaptée pour, pendant des séances de mesure d'impédance, mesurer des signaux d'impédance obtenus par une configuration d'électrodes qui est positionnée de telle manière que lesdits signaux d'impédance reflètent essentiellement des mouvements de paroi septale, les électrodes de ladite configuration d'électrodes pouvant être connectées audit dispositif médical implantable ;
une unité de détection de pics d'impédance (46) adaptée pour traiter lesdits signaux d'impédance afin de déterminer une morphologie de signal d'impédance et de détecter des pics d'amplitude d'impédance dans ladite morphologie de signal d'impédance ; et
**caractérisé par :**
une unité de détermination d'indice de synchronicité (48) adaptée pour :
déterminer un premier indice de synchronicité indiquant un degré de synchronicité sur la base de pics d'impédance détectés dans une fenêtre temporelle correspondant à une systole, au moins deux pics d'impédance indiquant une désynchronicité accrue ;
déterminer un second indice de synchronicité indiquant un degré de synchronicité sur la base de pics d'impédance détectés dans une fenêtre temporelle correspondant à une diastole, au moins deux pics d'impédance indiquant une désynchronicité accrue ;
associer un poids respectivement auxdits premier et second indices de synchronicité ; et
optimiser un indice total de synchronicité sur la base desdits premier et second indices de synchronicité afin d'obtenir un indice total minimisé ou optimiser lesdits premier et second indices de synchronicité séparément afin d'obtenir un indice minimisé respectivement en systole et en diastole.

2. Dispositif médical implantable selon l'une quelconque des revendications précédentes, ladite unité de détection de pics d'impédance étant adaptée pour calculer une première dérivée dudit signal d'impédance et pour détecter des points de minimums locaux et/ou maximums locaux de ladite première dérivée en tant que pics d'impédance.

3. Dispositif médical implantable selon la revendication 1 ou 2, dans lequel ladite unité de détermination d'indice de synchronicité est adaptée pour déterminer un indice de synchronicité sur la base de :
- un écart de pic entre deux pics détectés dans ladite fenêtre temporelle, un écart de pic accru correspondant à une valeur accrue de l'indice de synchronicité, et/ou
- un nombre de pics détectés dans ladite fenêtre temporelle prédéfinie, un nombre accru de pics correspondant à une valeur accrue de l'indice de synchronicité, et/ou
- une surface totale de pics de pics détectés dans ladite fenêtre temporelle prédéfinie, mesurée au-dessus d'un seuil prédéfini, une surface accrue de pics correspondant à une valeur accrue de l'indice de synchronicité ; et/ou
- une variabilité de l'amplitude de pics détectés, une variabilité d'amplitude accrue correspondant à une valeur accrue de l'indice de synchronicité ; et/ou
- une valeur absolue d'une amplitude totale de pic des pics détectés dans ladite fenêtre temporelle prédéfinie, une amplitude totale de pic accrue correspondant à une valeur accrue de l'indice de synchronicité.

4. Dispositif médical implantable selon l'une quelconque des revendications précédentes, comportant en outre un capteur de fréquence respiratoire adapté pour détecter un cycle respiratoire dudit patient, ladite unité de détermination de synchronicité étant adaptée pour déterminer ledit indice de synchronicité en synchronisme avec un événement dudit cycle respiratoire dudit patient ou en tant que valeur moyenne sur un nombre prédéfini de cycles respiratoires.

5. Dispositif médical implantable selon l'une quelconque des revendications précédentes, comportant en outre un capteur de posture corporelle adapté pour détecter une posture corporelle dudit patient, ladite unité de détermination de synchronicité étant adaptée pour déterminer ledit indice de synchronicité en synchronisme avec une posture corporelle prédéfinie dudit patient ou en tant que valeur moyenne de l'indice de synchronicité d'au moins deux postures corporelles.

6. Dispositif médical implantable selon l'une quelconque des revendications précédentes, comprenant en outre une unité de détermination de délai W adaptée pour effectuer une procédure d'optimisation, ledit générateur d'impulsions de stimulation étant commandé pour, sur la base dudit indice de synchronicité, ajuster de manière itérative un intervalle W de façon à minimiser ledit indice de synchronicité dans ladite fenêtre temporelle prédéfinie pour obtenir des contractions de ventricules essentiellement synchronisées.

7. Dispositif médical implantable selon l'une quelconque des revendications précédentes, comprenant en outre un circuit d'électrocardiogramme endocavitaire adapté pour capter des signaux d'électrocardiogramme endocavitaire de cycles cardiaques consécutifs et pour détecter des événements cardiaques dans lesdits cycles cardiaques au moyen desdits signaux d'électrocardiogramme endocavitaire ; et
dans lequel ledit circuit de détermination de mesure de synchronicité est adapté pour déterminer la fenêtre temporelle prédéfinie sur la base desdits événements cardiaques détectés et desdits signaux d'électrocardiogramme endocavitaire et/ou signaux d'impédance.

8. Dispositif médical implantable selon l'une quelconque des revendications précédentes, dans lequel ladite configuration d'électrodes comporte au moins une première paire d'électrodes présentant une électrode annulaire et une électrode pointue agencées dans une dérivation médicale positionnée dans le ventricule droit, ou une première électrode positionnée adjacente au septum dans le ventricule droit et une seconde électrode positionnée dans une veine coronaire sur le ventricule gauche ou la boîte.

9. Système destiné à optimiser des positions de dérivation et/ou d'électrode comportant un dispositif médical implantable (10), ledit dispositif comportant
un générateur d'impulsions de stimulation (42) adapté pour produire des impulsions régulatrices de stimulation cardiaque et qui peut être connecté à au moins une dérivation médicale pour transmettre des impulsions de stimulation à du tissu cardiaque dudit coeur ;
une unité de mesure d'impédance (41) adaptée pour, pendant des séances de mesure d'impédance, mesurer des signaux d'impédance obtenus sur une première configuration d'électrodes qui est positionnée de telle manière que lesdits signaux d'impédance reflètent essentiellement des mouvements de paroi septale, les électrodes de ladite configuration d'électrodes pouvant être connectées audit dispositif ;
une unité de détection de pics d'impédance (46) adaptée pour traiter lesdits signaux d'impédance afin de déterminer une morphologie de signal d'impédance et de détecter des pics d'amplitude d'impédance dans ladite morphologie de signal d'impédance ;
une unité de détermination d'indice de synchronicité (48) adaptée pour déterminer un indice de synchronicité indiquant un degré de synchronicité sur la base de pics d'impédance détectés pour ladite première configuration d'électrodes,
**caractérisé en ce que :**
ladite unité de détermination d'indice de synchronicité est adaptée pour :
déterminer un premier indice de synchronicité indiquant un degré de synchronicité sur la base de pics d'impédance détectés dans une fenêtre temporelle correspondant à une systole, au moins deux pics d'impédance indiquant une désynchronicité accrue ;
déterminer un second indice de synchronicité indiquant un degré de synchronicité sur la base de pics d'impédance détectés dans une fenêtre temporelle correspondant à une diastole, au moins deux pics d'impédance indiquant une désynchronicité accrue ;
associer un poids respectivement auxdits premier et second indices de synchronicité ; et
optimiser un indice total de synchronicité sur la base desdits premier et second indices de synchronicité afin d'obtenir un indice total minimisé ou optimiser lesdits premier et second indices de synchronicité séparément afin d'obtenir un indice minimisé respectivement en systole et en diastole
une unité de détermination de délai W adaptée pour effectuer une procédure d'optimisation, ledit générateur d'impulsions de stimulation étant commandé pour, sur la base dudit indice de synchronicité, ajuster de manière itérative un intervalle W de façon à minimiser ledit indice de synchronicité dans ladite fenêtre temporelle prédéfinie ; et
une unité de commande extérieure (90) pouvant être connectée audit dispositif médical implantable et qui est adaptée pour :
ordonner audit dispositif médical implantable d'obtenir un indice de synchronicité pour au moins une seconde configuration d'électrodes ;
comparer ledit indice de synchronicité minimal pour chaque configuration afin d'identifier un indice de synchronicité minimal global ; et
sélectionner la configuration d'électrodes qui est associée à l'indice de synchronicité minimal.

10. Système selon la revendication 9, dans lequel ledit dispositif médical implantable est adapté conformément à l'une quelconque des revendications 2 à 5, ou 7.

11. Analyseur de stimulation destiné à optimiser des positions de dérivation et/ou d'électrode, qui peut être connecté à au moins une dérivation médicale implantable dans un coeur d'un patient, ledit analyseur comportant
un générateur d'impulsions de stimulation adapté pour produire des impulsions régulatrices de stimulation cardiaque et qui peut être connecté à au moins une dérivation médicale pour transmettre des impulsions de stimulation à du tissu cardiaque dudit coeur ;
une unité de mesure d'impédance adaptée pour, pendant des séances de mesure d'impédance, mesurer des signaux d'impédance obtenus sur une configuration d'électrodes et/ou une configuration de dérivations, qui est/sont positionnée(s) de telle manière que lesdits signaux d'impédance reflètent essentiellement des mouvements de paroi septale, les électrodes de ladite configuration d'électrodes pouvant être connectées audit dispositif ;
une unité de détection de pics d'impédance adaptée pour traiter lesdits signaux d'impédance afin de déterminer une morphologie de signal d'impédance et de détecter des pics d'amplitude d'impédance dans ladite morphologie de signal d'impédance ;
une unité de détermination d'indice de synchronicité adaptée pour déterminer un indice de synchronicité indiquant un degré de synchronicité sur la base de pics d'impédance détectés pour ladite configuration d'électrodes,
**caractérisé en ce que** :
ladite unité de détermination d'indice de synchronicité est adaptée pour :
déterminer un premier indice de synchronicité indiquant un degré de synchronicité sur la base de pics d'impédance détectés dans une fenêtre temporelle correspondant à une systole, au moins deux pics d'impédance indiquant une désynchronicité accrue ;
déterminer un second indice de synchronicité indiquant un degré de synchronicité sur la base de pics d'impédance détectés dans une fenêtre temporelle correspondant à une diastole, au moins deux impédances indiquant une désynchronicité accrue ;
associer un poids respectivement auxdits premier et second indices de synchronicité ; et
optimiser un indice total de synchronicité sur la base desdits premier et second indices de synchronicité afin d'obtenir un indice total minimisé ou optimiser lesdits premier et second indices de synchronicité séparément afin d'obtenir un indice minimisé respectivement en systole et en diastole ;
une unité de détermination de délai W adaptée pour effectuer une procédure d'optimisation, ledit générateur d'impulsions de stimulation étant commandé pour, sur la base dudit indice de synchronicité, ajuster de manière itérative un intervalle W de façon à minimiser ledit indice de synchronicité dans ladite fenêtre temporelle prédéfinie ; et
une unité de commande adaptée pour :
comparer ledit indice de synchronicité minimal pour des configurations différentes d'électrodes et/ou de dérivations afin d'identifier un indice de synchronicité minimal global ; et
indiquer la configuration d'électrodes qui est associée à l'indice de synchronicité minimal.
